# EUROPÄISCHE PATENTSCHRIFT

(11) **EP 1 329 232 B1**
(45) Veröffentlichungstag und Bekanntmachung des Hinweises auf die Patenterteilung: **19.04.2006**
(21) Anmeldenummer: 03000283.6
(22) Anmeldetag: 09.01.2003
(51) Int. Cl.: A61M 5/145

(54) **Spritzenpumpe mit Kolbenbremse**
Syringe pump with piston brake
Pompe pour seringue avec un frein agissant sur le piston de la seringue

(30) Priorität: 22.01.2002 DE 20200885 U
(43) Veröffentlichungstag der Anmeldung: 23.07.2003
(73) Patentinhaber: B. BRAUN MELSUNGEN AG, 34212 Melsungen (DE)
(72) Erfinder: Gerlach, Hans-Josef, 34431 Marsberg (DE); Wildner, René, 34212 Melsungen (DE); Wolfram, Berthold, 34212 Melsungen (DE)
(74) Vertreter: Selting, Günther

(56) Entgegenhaltungen:
- EP-A- 0 566 825
- EP-A- 1 195 172

## Beschreibung

Die Erfindung betrifft eine Spritzenpumpe mit Kolbenbremse zum kontrollierten Ausdrücken medizinischer Spritzen.

Aus EP 0 566 825 A1 ist eine Spritzenpumpe mit Kolbenbremse bekannt, die einen ersten Halter zum Festlegen des Spritzenzylinders einer Spritze und einen linear bewegbaren zweiten Halter zum Verschieben der Kolbenstange der Spritze aufweist.

An der Kolbenstange greift eine an ortsfester Position vorgesehene Kolbenbremse an, die bei eingelegtem Spritzenzylinder, die Kolbenstange blockiert, solange die Kolbenstange noch nicht von dem zweiten Halter ergriffen wurde. Dadurch werden unbeabsichtigte Verschiebungen der Kolbenstange durch Saugwirkung oder durch mechanisches Bewegen vor Infusionsbeginn vermieden. Ein Sichern der Kolbenstange nach einem Spritzenwechsel ist insbesondere bei kleinen Spritzen, die geringe Reibkräfte aufweisen, wichtig.

Bekannt sind ferner Spritzenpumpen, die einen automatischen Spritzenwechsel durchführen können. Hierbei ist ein Spritzenbügel vorgesehen, der nach dem Einlegen einer Spritze diese Spritze abtastet und die Spritzengröße ermittelt. Aufgrund der Spritzengröße werden automatisch die Vorschubgeschwindigkeit und andere Parameter eingestellt.

Der Erfindung liegt die Aufgabe zugrunde, eine Spritzenpumpe mit Kolbenbremse zu schaffen, die mit einer vereinfachten Abstützung der Spritze in der Spritzenposition auskommt.

Die Lösung dieser Aufgabe erfolgt erfindungsgemäß mit den im Anspruch 1 angegebenen Merkmalen.

Bei der erfindungsgemäßen Spritzenpumpe ist das Bremselement auf der der Antriebsstange abgewandten Seite der Spritzenposition angeordnet, also auf der Außenseite. Das Bremselement ist in Richtung auf ein auf der Seite der Antriebsstange angeordnetes Spritzenlager bewegbar, um die Bremswirkung auszuüben. Dies hat zur Folge, dass sich das Spritzenlager auf der Seite der Antriebsstange befindet, welche die Innenseite bildet. Gegen dieses Spritzenlager können die Spritzen unterschiedlicher Größe angelegt werden, um eine zum Ausdrücken geeignete definierte Position einzunehmen.

Gemäß einer bevorzugten Weiterbildung der Erfindung ist das Bremselement an einem Spritzenbügel vorgesehen, welcher quer zu einer in der Spritzenposition befindlichen Spritze bewegbar ist. Ein derartiger Spritzenbügel greift üblicherweise nur an dem Spritzenzylinder an. Er dient als Halter, der den Spritzenzylinder gegen Herausnehmen aus der Spritzenposition sichert. Erfindungsgemäß ist dieser Spritzenbügel zusätzlich mit einem Bremselement ausgestattet, das auf die Kolbenstange einwirkt, welche aus dem Spritzenzylinder herausragt. Ein Vorteil dieser Maßnahme besteht darin, dass die Kolbenbremse einen räumlichen Bezug zum Spritzenzylinder hat, d.h. zur Aktivierung und Deaktivierung der Kolbenbremse brauchen jeweils nur sehr kleine Verschiebebewegungen des Bremselements ausgeführt zu werden. Nach dem Schließen des Spritzenbügels und dem Erkennen einer gültigen Spritze greift die Kolbenbremse automatisch solange, bis der motorisch angetriebene Antriebskopf die Kolbenplatte der Spritze erkannt und verriegelt hat. Danach wird die Kolbenbremse durch Verschieben des Bremselements gelöst, wobei der Eingriff des Spritzenbügels an dem Spritzenzylinder jedoch nicht aufgegeben wird.

Bei einer bevorzugten Ausführungsform der Erfindung ist vorgesehen, dass ein Linearantrieb für das Bremselement sich unter der Spritzenposition hindurch erstreckt, wobei ein Motor auf der Seite und der Spritzenbügel auf der anderen Seite der Antriebsstange angeordnet ist. Der Linearantrieb bewirkt die Verstellung des Bremselements von der Seite der Antriebsstange aus, wobei die Spritze gegen das Spritzenlager gezogen wird, das sich ebenfalls auf der Seite der Antriebsstange befindet.

Vorzugsweise ist das Bremselement an einem Spritzenbügel vorgesehen, wobei ein Teil des Spritzenbügels an dem Spritzenzylinder und das Bremselement an der Kolbenstange angreift. Auf diese Weise können die Funktionen von Spritzenbügel und Bremselement in einem Teil vereinigt werden.

Gemäß einer bevorzugten Ausgestaltung der Erfindung ist ein Betätigungsorgan für das Bremselement auf einem Schlitten angeordnet, der mit dem Spritzenbügel verbunden ist, und es ist ein die Position des Schlittens feststellender Sensor zur Ermittlung der Spritzengröße vorgesehen. Wenn der Spritzenbügel zum Einlegen einer Spritze manuell herausgezogen und anschließend gegen die Spritze gesetzt wird, bewegt sich der Schlitten, der das Betätigungsorgan für das Bremselement trägt, zusammen mit dem Spritzenbügel. Wenn die Spritze sich in der Spritzenposition befindet, liefert der Sensor ein Positionssignal, das die Position des Spritzenbügels und somit die Größe (den Durchmesser) der eingelegten Spritze angibt.

Vorzugsweise ist der Schlitten mit dem Spritzenbügel durch ein Rohr verbunden, durch welches eine axialbewegbare Stange zur Verstellung des Bremselements relativ zum Spritzenbügel verläuft. Dadurch ergibt sich ein besonders einfacher Verstellmechanismus für das Bremselement in Bezug auf den Spritzenbügel.

Um die Spritze beim Einlegen in die Spritzenpumpe auch in axialer Richtung zur Spritze definiert zu positionieren, ist gemäß einer bevorzugten Weiterbildung der Erfindung eine an einem Spritzenflügel der Spritze angreifende Positioniervorrichtung vorgesehen, die mit dem Spritzenbügel derart gekoppelt ist, dass sie den Spritzenflügel axial gegen eine Anschlagwand drückt, wenn der Spritzenbügel sich einer eingelegten Spritze radial nähert.

Die Erfindung betrifft ferner eine Spritzenpumpe mit den Merkmalen des Patentanspruchs 8. Hierbei ist das die Kolbenbremse bildende Bremselement an einem Spritzenbügel vorgesehen, der den Spritzenzylinder radial gegen ein Spritzenlager drückt.

Im folgenden wird ein Ausführungsbeispiel der Erfindung unter Bezugnahme auf die Zeichnungen näher erläutert.

Es zeigen:
- Fig. 1: eine Draufsicht auf einen Teil einer Spritzenpumpe bei herausgezogenem Spritzenbügel und in der Freigabeposition befindlicher Sperreinrichtung,

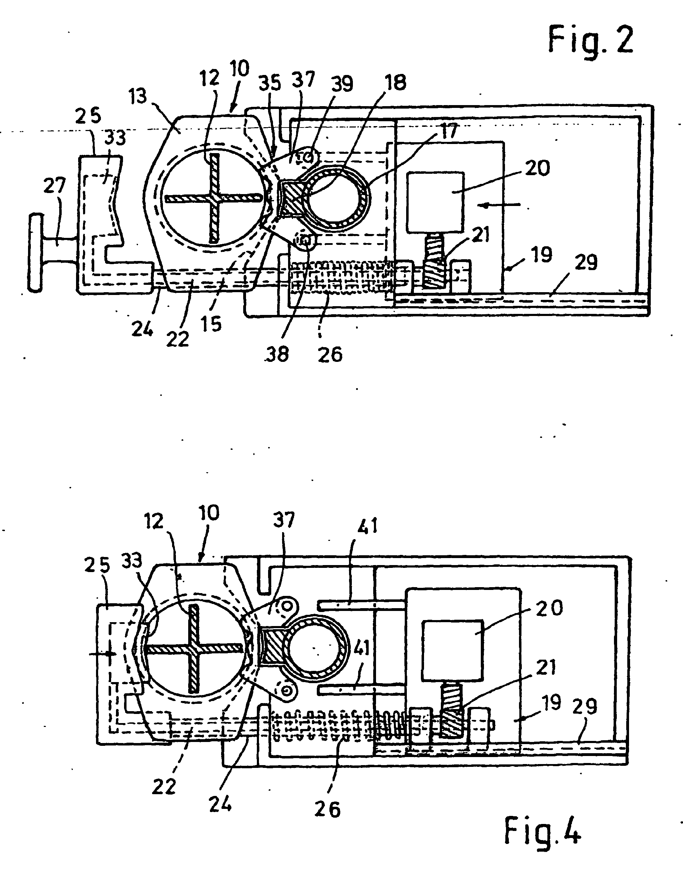

- Fig. 2: einen Schnitt entlang der Linie II-II von Figur 1,
- Fig. 3: in gleicher Darstellung wie Figur 1 den Zustand bei ordnungsgemäß eingelegter Spritze und geschlossenem Spritzenbügel,

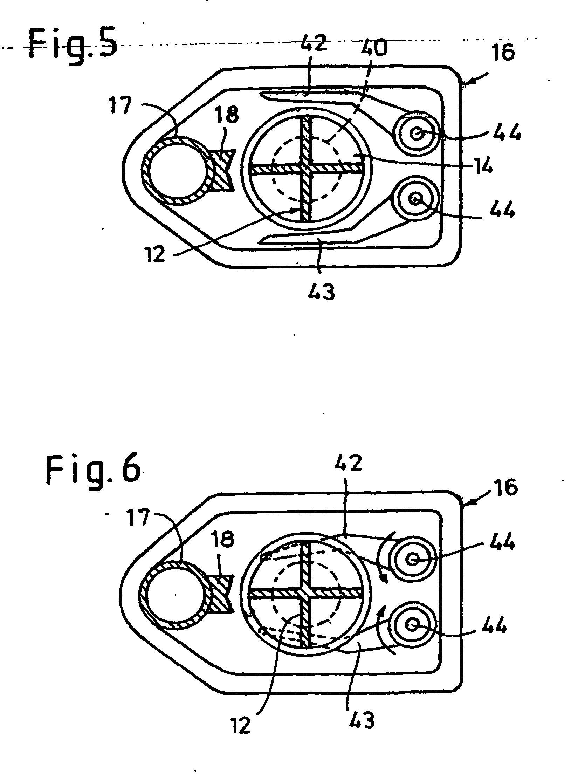

- Fig. 4: einen Schnitt entlang der Linie IV-IV von Figur 3,
- Fig. 5: einen Schnitt entlang der Linie V-V von Figur 3 bei geöffneten Armen des Antriebskopfes,
- Fig. 6: den Antriebskopf von Figur 5, wobei die Arme mit der Kolbenstange verriegelt sind,
- Fig. 7: einen Schnitt entlang der Linie VII-VII von Figur 3, und
- Fig. 8: einen Ausschnitt aus Figur 1, wobei die Sperreinrichtung für den Spritzenbügel in der Sperrposition ist.

Die Spritzenpumpe ist generell so ausgebildet, wie diejenige von EP 0 566 825 A1, so dass eine generelle Beschreibung des gesamten Geräts entbehrlich ist.

Die Spritzenpumpe dient zum Ausdrücken einer Spritze 10, welche einen Spritzenzylinder 11 und eine Kolbenstange 12 aufweist.

Der Spritzenzylinder 11 ist an seinem offenen Ende mit einem abstehenden Spritzenflügel 13 versehen und die Kolbenstange weist an ihrem Ende eine Kolbenplatte 14 auf. Die Kolbenstange 12 hat ein kreuzförmiges Profil aus rechtwinklig angeordneten Rippen.

Die Spritze 10 wird in der dargestellten Spritzenposition P gegen ein seitliches Spritzenlager 15 gesetzt, und zwar so, dass der Spritzenflügel 13 eine vorgegebene axiale Position einnimmt. In diesem Zustand ist die Kolbenstange 12 aus dem Spritzenzylinder 11 herausgefahren und der Spritzenzylinder 11 ist mit Flüssigkeit gefüllt.

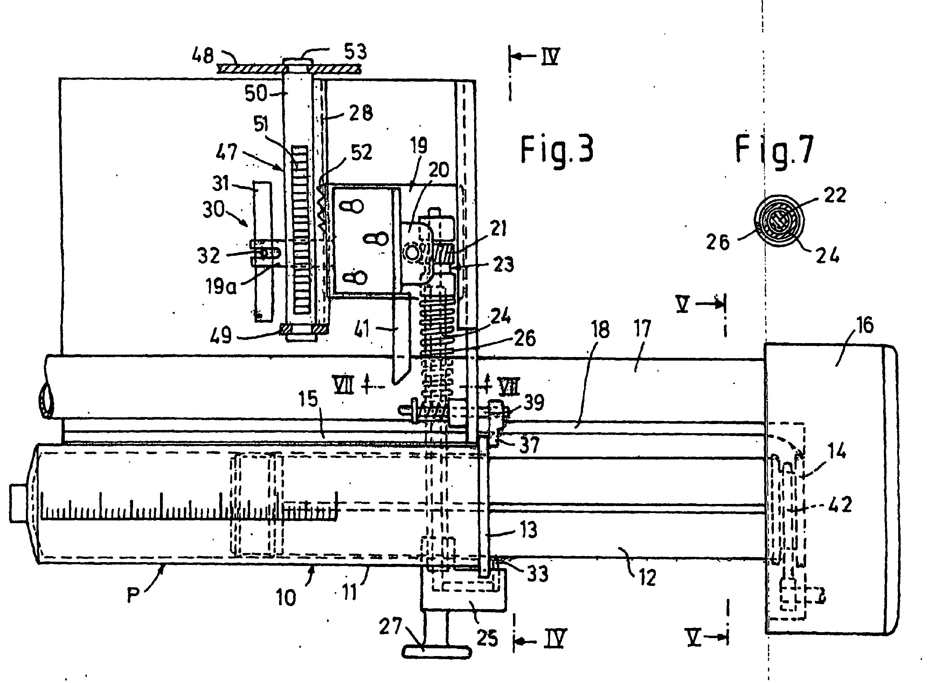

Zum Bewegen der Kolbenstange 12 ist an der Spritzenpumpe ein linear bewegbarer Antriebskopf 16 vorgesehen, der an einer langgestreckten Antriebsstange 17 angebracht ist. Der Antriebskopf kann an die Kolbenplatte 14 herangefahren werden, um diese aufzunehmen und zu verriegeln. Er kann dann weiter gemäß Fig. 3 nach links bewegt werden, um den Inhalt der Spritze 10 auszudrücken. An der Seite der Antriebsstange 17 befindet sich ein Stützlager 18, gegen das sich bei Eingriff der Kolbenbremse die Kolbenplatte 14 abstützen kann.

Auf der Seite der Antriebsstange 17 ist auf einem Schlitten 19 ein Motor 20 mit senkrechter Achse angeordnet. Dieser Motor 20 treibt über ein Schneckenrad ein auf dem Schlitten 19 gelagertes Zahnrad 21 mit Schrägverzahnung. Das Zahnrad 21 ist mit einer Gewindebohrung versehen, die mit einem Gewinde einer Spindelstange 22 in Eingriff steht. Durch Drehen des Zahnrades 21 wird die Spindelstange 22, die gegen Mitdrehen gesichert ist, axial verschoben. Damit bildet die Spindelstange 22 einen Linearantrieb 23.

Von dem Schlitten 19 erstreckt sich ein Rohr 24, durch welches die Spindelstange 22 verläuft, quer zur Längsrichtung der Spritze 10 unter der Antriebsstange 17 hindurch. Auf der gegenüberliegenden Seite der Spritze 10 ist an dem Ende des Rohres 24 ein Spritzenbügel 25 befestigt. Auf dem Rohr 24 sitzt eine schraubenförmige Feder 26, die an dem zum Gehäuse der Spritzenpumpe gehörenden Spritzenlager 15 abgestützt ist und den Schlitten 19 von der Spritzenposition fortdrückt. Da andererseits das Rohr 24 mit dem Schlitten 19 verbunden ist und auf dem Ende des Rohres 24 der Spritzenbügel 25 sitzt, wird der Spritzenbügel gegen die in der Spritzenposition P befindliche Spritze 10 gedrückt und drückt damit die Spritze gegen das Spritzenlager 15. Der Spritzenbügel 25 ist um die Achse des Rohres 24 drehbar. Er weist einen Griffknopf 27 auf, mit dem er manuell von der Spritze fortgezogen und auch gedreht werden kann.

Der Schlitten 19 ist in Führungsschienen 28,29 quer zur Längsrichtung der in der Spritzenposition P befindlichen Spritze 10 geführt. Neben dieser Linearführung ist ein Sensor 30 aus einem Potentiometer 31 angeordnet. Der Abgriff 32 des Potentiometers 31 wird mit einem Schieber 19a, der mit dem Schlitten 19 verbunden ist, verstellt. Auf diese Weise gibt der elektrische Widerstand des Potentiometers 31 die Stellung des Schlittens 19 und somit auch die Stellung des Spritzenbügels 25 an. Das Signal des Sensors 30 gibt somit Aufschluss über die Spritzengröße bzw. den Spritzendurchmesser bei geschlossenem Spritzenbügel 25.

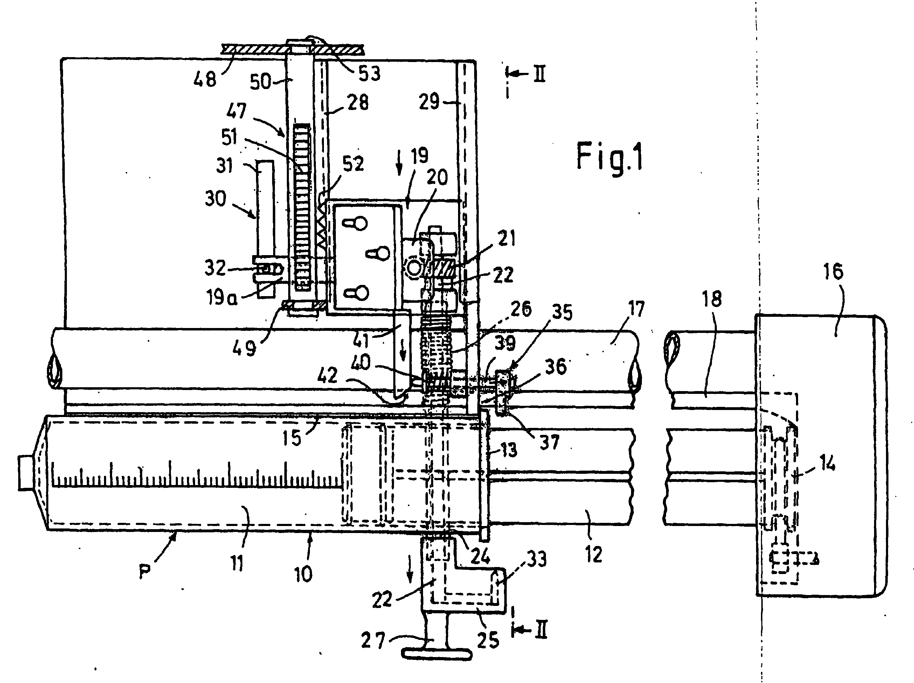

Die Spindelstange 22 ragt in den Spritzenbügel 25 hinein und trägt innerhalb des Spritzenbügels 22 ein Bremselement 33 in Form einer Schneide, deren Spitze der Spritze 11 radial zugewandt ist. Durch Betätigung des Motors 20 wird das Bremselement 33 zwischen einer Rückzugsposition (Figur 1) und einer aktiven Bremsposition (Figur 3) bewegt. In der Bremsposition greift das Bremselement 33, welches über den Spritzenflügel 13 hinweggreift, an der Kolbenstange 12 an, um Verschiebungen der Kolbenstange 12 in Bezug auf den Spritzenzylinder 11 zu blockieren.

Die Spritzenpumpe ist ferner mit einer axialen Positioniervorrichtung 35 versehen, die den Spritzenflügel 13 axial gegen eine Anschlagwand 36 drückt. Die Positioniervorrichtung 35 weist einen Schieber 37 auf, der an zwei Stangen 38 axial zur Spritze 10 geführt ist. Der Schieber 37 steht in den Weg des Spritzenflügels 13 vor. Er ist an zwei parallelen Stangen 38,39 befestigt, die packsparallel zu der Spritze 10 verlaufen und von jeweils einer Feder 40 nach vorne, also in Richtung auf das Auslassende des Spritzenzylinders 11, vorgespannt sind. Die rückwärtigen Enden der Stangen 39 werden jeweils durch ein mit dem Schlitten 19 verbundenes Steuerelement 41 gesteuert, da das eine nockenwirkende Schrägfläche 42 aufweist. Wenn der Schlitten 19 in Richtung auf die Spritzenposition P bewegt wird, schieben die Steuerelemente 41 die Platte 37 nach hinten, also von der Anschlagwand 36 fort, sodass der Spritzenflügel 13 dazwischengesetzt werden kann. Wird anschließend der Spritzenbügel 25 losgelassen, sodass er sich gegen den Spritzenzylinder 11 legt, wird die Platte 37 unter der Wirkung der Federn 40 nach vorne gezogen, wodurch sie den Spritzenflügel 13 gegen die Anschlagwand 36 drückt und somit eine exakte axiale Positionierung des Spritzenzylinders 11 vornimmt.

Der Antriebskopf 16 enthält einen Kolbenplattensensor 40, der auf die Anwesenheit einer Kolbenplatte 14 reagiert und dadurch dem Gerät mitteilt, dass der Antriebskopf 16 an die Kolbenplatte 14 einer eingelegten Spritze herangefahren ist. Der Kolbenplattensensor 40 ist in einer Vertiefung des Antriebskopfs 16 angeordnet. In der Vertiefung sind außerdem gesteuerte Arme 42,43 vorgesehen, die um jeweils einen Gelenkzapfen 44, schwenkbar sind und aufeinander zu bewegt werden können, um die Kolbenplatte 14 an dem Antriebskopf zu blockieren.

Zum Einlegen einer Spritze 10 in die Spritzenpumpe wird der Spritzenbügel 25 an dem Griffknopf 27 herausgezogen. Dabei wird gleichzeitig die Positioniervorrichtung 35 in die Öffnungsstellung gebracht. Nun kann der Spritzenzylinder 11 eingelegt werden. Danach wird der Spritzenbügel 25 von außen her gegen den Spritzenzylinder 11 gelegt. Die Feder 26 bewirkt, dass der Spritzenbügel 25 den Spritzenzylinder 11 gegen das Spritzenlager 15 drückt. Während des Anlegens des Spritzenbügels 25 an den Spritzenzylinder 11 drückt die Positioniervorrichtung 35 mit ihrer Platte 37 den Spritzenflügel 13 gegen die Anschlagwand 36. Nach dem Loslassen des Spritzenbügels 25 ist die Spritze exakt positioniert. Der Sensor 30 stellt anhand der Position, die der Schlitten 19 dann einnimmt, die Spritzengröße fest und liefert ein entsprechendes Signal an die Steuereinrichtung. Bei der Ermittlung der Vorschubgeschwindigkeit wird die Spritzengröße berücksichtigt.

Beim Loslassen des Spritzenbügels 25 wird der Motor 20 in Funktion gesetzt, sodass die Spitze des Bremselements 33 aus dem Spritzenbügel 25 herausgefahren und gegen die Kolbenstange 12 gedrückt wird. Dadurch wird die Kolbenstange 12 in Bezug auf den Spritzenzylinder 11 fixiert.

Dann wird automatisch die Antriebsstange 17 derart bewegt, dass der Antriebskopf 16 an die Kolbenplatte 14 heranfährt. Wenn der Kolbensensor 40 das Anliegen der Kolbenplatte feststellt, werden die Arme 42,43 betätigt, um die Kolbenplatte im Antriebskopf 16 zu verriegeln. Dann wird der Motor 20 in Rückwärtsrichtung betätigt, um das Bremselement 33 von der Kolbenstange 12 zu entfernen. Im folgenden kann dann der Antriebskopf 16 zum Ausdrücken der Spritze 10 bewegt werden.

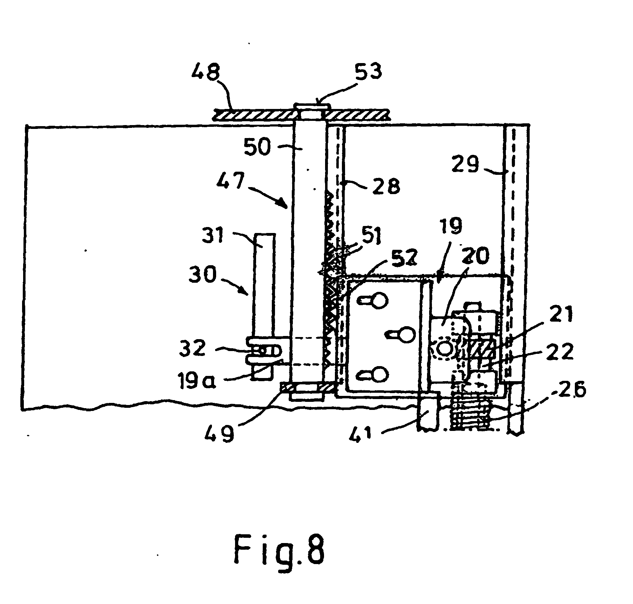

Es besteht ferner die Möglichkeit, eine Zwangsbegrenzung für die Bewegung des Schlittens 19 vorzusehen, um die eingelegte Spritze am Gerät zu sichern. Eine solche Sicherung der Spritze kann insbesondere bei der PCA (Patientenkontrollierte Analgesie) zweckmäßig sein. Hierbei handelt es sich um eine Schmerzbehandlung. Die Verriegelung verhindert, dass dem Patienten durch eigene Manipulation oder durch Manipulation von Dritten eine zu hohe Medikamentendosis zugeführt wird. Die Zwangsbegrenzung erfolgt durch eine Sperreinrichtung 47, die einen parallel zum Schlitten 19 an Gehäusewänden 48,49 drehbar gelagerten Schaft 50 aufweist, der an einer Seite eine zahnstangenartige Verzahnung 51 aufweist. Diese Verzahnung 51 kann durch Drehen des Schafts 50 in ein am Schlitten 19 befindliches Zahnsegment 52 eingreifen. Wenn die Verzahnungen in Eingriff sind (Figur 8), ist der Schlitten 19 in seiner Bewegung gesperrt. Das Drehen des Schafts 50 ist nur mit einem Spezialschlüssel möglich, der in eine an der Stirnwand 53 vorgesehene (nicht dargestellte) Profilöffnung eingesteckt wird.

## Patentansprüche

1. Spritzenpumpe mit Kolbenbremse, mit einem Spritzenhalter zum Halten eines Spritzenzylinders (11) einer eingelegten Spritze (10) in einer Spritzenposition (P), einem während des Gebrauchs der Spritzenpumpe an einer Kolbenplatte (14) der Spritze (10) angreifenden Antriebskopf (16), der von einer parallel zu der eingelegten Spritze verlaufenden Antriebsstange (17) getragen ist, und einem quer zur Längsrichtung der Spritze gegen die Kolbenstange (12) bewegbaren Bremselement (33),
**dadurch gekennzeichnet,**
**dass** das Bremselement (33) auf der der Antriebsstange (17) abgewandten Seite der Spritzenposition (P) angeordnet ist und in Richtung auf ein auf der Seite der Antriebsstange (17) angeordnetes Spritzenlager (15) drückt.

2. Spritzenpumpe nach Anspruch 1, **dadurch gekennzeichnet, dass** das Bremselement (33) an einem Spritzenbügel (25) vorgesehen ist, welcher quer zu einer in der Spritzenposition (P) befindlichen Spritze (10) bewegbar ist.

3. Spritzenpumpe nach Anspruch 1 oder 2, **dadurch gekennzeichnet, dass** ein Linearantrieb (23) für das Bremselement (33) sich unter der Spritzenposition (P) erstreckt, wobei ein Betätigungsorgan (20) auf der einen Seite der Antriebsstange (17) und der Spritzenbügel (25) auf der anderen Seite angeordnet ist.

4. Spritzenpumpe nach Anspruch 2, **dadurch gekennzeichnet, dass** ein Betätigungsorgan (20) für das Bremselement (33) auf einem Schlitten (19) angeordnet ist, der mit dem Spritzenbügel (25) gekoppelt ist, und dass ein die Position des Schlittens (19) feststellender Sensor (30) zur Ermittlung der Spritzengröße vorgesehen ist.

5. Spritzenpumpe nach Anspruch 4, **dadurch gekennzeichnet, dass** der Schlitten (19) mit dem Spritzenbügel (25) durch ein Rohr (24) verbunden ist, durch welches eine axialbewegbare Stange (22) zur Verstellung des Bremselements (33) relativ zum Spritzenbügel (25) verläuft.

6. Spritzenpumpe nach einem der Ansprüche 1-5, **dadurch gekennzeichnet, dass** das Bremselement (33) an einem Spritzenbügel (25) vorgesehen ist, wobei ein Teil des Spritzenbügels (25) an dem Spritzenzylinder (11) und das Bremselement (33) an der Kolbenstange (12) angreift.

7. Spritzenpumpe nach einem der Ansprüche 2-6, **dadurch gekennzeichnet, dass** eine an einem Spritzenflügel (13) angreifende Positioniereinrichtung (35) vorgesehen ist, die mit dem Spritzenbügel (25) derart gekoppelt ist, dass sie den Spritzenflügel (13) gegen eine Anschlagwand (36) drückt, wenn der Spritzenbügel (25) sich einer eingelegten Spritze (10) radial nähert.

8. Spritzenpumpe nach einem der Ansprüche 2-7, **dadurch gekennzeichnet, dass** eine den Spritzenbügel (25) in seiner Position blockierende lösbare Sperreinrichtung (47) vorgesehen ist.

9. Spritzenpumpe nach Anspruch 8, **dadurch gekennzeichnet, dass** die Sperreinrichtung (47) an dem Schlitten (19) angreift.

10. Spritzenpumpe nach Anspruch 8 oder 9, **dadurch gekennzeichnet, dass** die Sperreinrichtung (47) einen drehbaren Schaft (50) mit zahnstangenartiger Verzahnung (51) aufweist.

11. Spritzenpumpe nach Anspruch 10, **dadurch gekennzeichnet, dass** der Schaft (50) eine mit einem Spezialschlüssel zusammenpassende Öffnung zum Betätigen und Lösen der Sperreinrichtung (47) aufweist.

## Claims

1. A syringe pump having a piston brake, comprising a syringe holder for holding a syringe cylinder (11) of a syringe (10) inserted in a syringe position (P), a drive head (16) engaging a piston plate (14) of the syringe (10) when the syringe pump is in use, the drive head (16) being carried by a drive rod (17) extending parallel to the inserted syringe, and a brake element (33) movable, transversely to the longitudinal direction of the syringe, against the piston rod (12),
**characterized in that**
the brake element (33) is arranged on the side of the syringe position (P) opposite the drive rod (17) and presses towards a syringe bearing (15) arranged on the drive rod (17) side.

2. The syringe pump according to claim 1, **characterized in that** the brake element (33) is provided on a syringe holding bar (25) which is movable transversely to a syringe (10) in the syringe position (P).

3. The syringe pump according to claim 1 or 2, **characterized in that** a linear drive (23) for the brake element (33) extends underneath the syringe position (P), an actuating means (20) being arranged on one side of the drive rod (17) and the syringe holding bar (25) being arranged on the other side.

4. The syringe pump according to claim 2, **characterized in that** an actuating means (20) for the brake element (33) is arranged on a carriage (19) coupled to the syringe holding bar (25), and that a sensor (30) detecting the position of the carriage (19) is provided for determining the syringe size.

5. The syringe pump according to claim 4, **characterized in that** the carriage (19) is connected via a tube (24) with the syringe holding bar (25), through which tube (24) extends an axially movable rod (22) for adjusting the brake element (33) relatively to the syringe holding bar (25).

6. The syringe pump according to one of claims 1-5, **characterized in that** the brake element (33) is provided on a syringe holding bar (25), a portion of the syringe holding bar (25) engaging the syringe cylinder (11), and the brake element (33) engaging the piston rod (12).

7. The syringe pump according to one of claims 2-6, **characterized in that** a positioning means (35) for engaging a wing-like syringe portion (13) is provided, the positioning means (35) being coupled to the syringe holding bar (25) such that it presses the wing-like syringe portion (13) against a stop wall (36) when the syringe holding bar (25) radially approaches an inserted syringe (10).

8. The syringe pump according to one of claims 2-7, **characterized in that** a releasable locking means (47) locking the syringe holding bar (25) in its position is provided.

9. The syringe pump according to claim 8, **characterized in that** the locking means (47) engages the carriage (19).

10. The syringe pump according to claim 8 or 9, **characterized in that** the locking means (47) comprises a rotatable shaft (50) having a rack-type toothing (51).

11. The syringe pump according to claim 10, **characterized in that** the shaft (50) comprises an opening matching a special wrench for actuating and releasing the locking means (47).

## Revendications

1. Pompe de seringue comportant un frein agissant sur le piston, un porte-seringue pour le maintien d'un cylindre de seringue (11) d'une seringue (10) à l'intérieur dans une position de seringue (P), une tête d'entraînement (16) agissant sur une plaque de piston (14) de la seringue (10) quand la pompe de seringue est en usage, tête qui est portée par une barre d'entraînement (17) parallèle à la seringue à l'intérieur, et un élément de freinage (33) pouvant se déplacer transversalement au sens longitudinal de la seringue contre la tige de piston (12),
**caractérisée en ce que**
l'élément de freinage (33) est disposé sur le côté de la position de seringue (P) opposé à la barre d'entraînement (17) et appuie en direction d'un palier de seringue (15) disposé sur le côté de la barre d'entraînement (17).

2. Pompe de seringue selon la revendication 1, **caractérisée en ce que** l'élément de freinage (33) est prévu sur un étrier de seringue (25) qui peut se déplacer transversalement à une seringue (10) se trouvant dans la position de seringue (P).

3. Pompe de seringue selon la revendication 1 ou 2, **caractérisée en ce qu'**un entraînement linéaire (23) destiné à l'élément de freinage (33) s'étend sous la position de seringue (P), un organe d'actionnement (20) étant disposé sur un côté de la barre d'entraînement (17) et l'étrier de seringue (25) sur l'autre côté.

4. Pompe de seringue selon la revendication 2, **caractérisée en ce qu'**un organe d'actionnement (20) destiné à l'élément de freinage (33) est disposé sur un chariot (19) qui est couplé à l'étrier de seringue (25) et un capteur (30), constatant la position du chariot (19), est prévu pour déterminer la taille de la seringue.

5. Pompe de seringue selon la revendication 4, **caractérisée en ce que** le chariot (19) est relié à l'étrier de seringue (25) par un tube (24) à travers lequel passe une barre (22) mobile axialement pour déplacer l'élément de freinage (33) par rapport à l'étrier de seringue (25).

6. Pompe de seringue selon l'une quelconque des revendications 1 à 5, **caractérisée en ce que** l'élément de freinage (33) est prévu sur un étrier de seringue (25), une partie de l'étrier de seringue (25) agissant sur le cylindre de seringue (11) et l'élément de freinage (33) sur la tige de piston (12).

7. Pompe de seringue selon l'une quelconque des revendications 2 à 6, **caractérisée en ce qu'**un dispositif de positionnement (35), qui agit sur une aile de la seringue (13) est prévu, dispositif qui est couplé à l'étrier de seringue (25) de manière à appuyer l'aile de seringue (13) contre une paroi de butée (36) lorsque l'étrier de seringue (25) se rapproche radialement d'une seringue (10) à l'intérieur.

8. Pompe de seringue selon l'une quelconque des revendications 2 à 7, **caractérisée en ce qu'**un dispositif d'arrêt (47), pouvant être débloqué, bloquant l'étrier de seringue (25) dans sa position, est prévu.

9. Pompe de seringue selon la revendication 8, **caractérisée en ce que** le dispositif d'arrêt (47) agit sur le chariot (19).

10. Pompe de seringue selon la revendication 8 ou 9, **caractérisée en ce que** le dispositif d'arrêt (47) présente une tige rotative (50) comportant une denture (51) de type crémaillère.

11. Pompe de seringue selon la revendication 10, **caractérisée en ce que** la tige (50) présente une ouverture adaptée à une clé spéciale permettant l'actionnement et le déblocage du dispositif d'arrêt (47).
